# EUROPEAN PATENT SPECIFICATION

(11) **EP 1 985 267 B1**
(45) Date of publication and mention of the grant of the patent: **25.12.2013**
(21) Application number: 07008537.8
(22) Date of filing: 26.04.2007
(51) Int. Cl.: A61F 5/451, B29C 49/20

(54) **Ostomy appliance collector**
Ostomievorrichtungskollektor
Collecteur d'appareil stomique

(43) Date of publication of application: 29.10.2008
(73) Proprietor: ConvaTec Technologies Inc., Las Vegas, NV 89169-6754 (US)
(72) Inventor: Veerapaneni, Bhavani, Skillmann, NJ 08558 (US); Alexander, Conor M., Norfolk, VA 23503 (US); Johnsen, Kenneth, Skillmann, NJ 08558 (US)
(74) Representative: Mays, Julie

(56) References cited:
- EP-A- 1 348 412
- EP-A- 1 595 683
- WO-A-90/07311
- GB-A- 1 346 499

## Description

### Field of the Invention

The present invention relates to a method of producing a collector device for collecting body waste in an ostomy appliance. The collector device may be of a type that is expandable from a compact form, in which the collector device may be stowed initially, to an expanded form, in which the collector device may be deployed for collecting the body waste. The collector device may be especially suitable for inclusion in a controlled evacuation ostomy appliance, but the invention is not limited exclusively to this. The term "ostomy" may include one or more of colostomy, ileostomy and urostomy.

### Background to the Invention

US Patents Nos. 6723079 and 6689111, US Published Patent Application 2004181197, Published PCT Application WO-A-02/058604, and DE-A-19921555 describe various designs of ostomy appliance that include expandable ostomy collection devices. Each collection device is tubular, and is provided initially in an axially collapsed form in which the collection device is stowed in the appliance. The collection device is expandable from its initial form, into an expanded form for collecting and containing body waste discharged from the stoma.

The first three of the above specifications describe ostomy appliances in the form of controlled evacuation devices that allow the user to control when a stomal discharge will take place. The appliances block or occlude the stoma, such that body waste is stored temporarily in the portion of the bowel leading to the stoma. When the blocking or occluding portion of the appliance is removed and the collection device expanded, body waste is discharged into the collection device. Controlled evacuation devices offer the potential for an ostomate to regain some control over bodily function, by controlling when a discharge of body waste will take place.

One of the desired features of all of the above appliances is that the appliance should preferably be compact, yet be able to provide a significant collection capacity. In one non-limiting form, the present invention may seek to provide a form of collection device that may be especially suitable for such appliances by enabling the collection device initially to have a highly compact form without interfering with other functional parts of the appliances, yet also provide a substantial collection volume for body waste, e.g., when the collection device is deployed to its expanded condition.

Reference may me made to EP-A-1595683, which is regarded as the closest prior art, disclosing a method and apparatus for forming an ostomy device component, for attaching a preformed (injection molded) first portion of the component to a second portion. The second portion is formed by blow molding a parison into intimate bonding contact with the preformed first portion. The preformed first portion is injection molded. Reference may also be made to WO-90/07311, EP-A-1348412 and GB-A-1346499.

### Summary of the Invention

The present invention is defined in the claims.

The following description illustrates a collection device for an ostomy appliance, at least a portion of the collection device being thermoformed.

The thermoformed portion is thermoformed from flexible plastics sheet or film.

The terms "thermoformed" or "thermoforming" may be used herein to refer to any process in which a pressure differential between opposite sides of the plastics sheet, may be used to draw or force the sheet against a shape defining Form. In one aspect of the invention, the thermoforming may be specifcally vacuum forming or pressure forming.

In one non-limiting one form, the invention can enable a collection device to be provided that (i) is formed from plastics sheet, and (ii) has a depth to transverse dimension ratio, or so-called "aspect ratio", (e.g. depth to diameter ratio) of at least 1:1. The ratio may be far greater than 1:1.

The process of thermoforming the collection device (portion) may be carried out using a male or female Form. The term "Form" may be used herein to refer to the shape-defining portion of the apparatus for thermoforming an article to a desired shape defined by the Form.

The collection device may be collapsed or collapsible into a compact form. For example, if the collection device has a tubular bag form, the collection device may be collapsed into a generally flat, or low profile, annulus.

The collection device may be expanded or distended from the compact form to a deployed form. The deployed form may be substantially the same shape in which the collection device is originally thermoformed. The use of thermoforming can enable the expanded shape to be well-defined and/or controlled, in contrast to, for example, a flexible collection device with little or no control over its shape as it fills.

The invention can enable production of a thin-walled tubular bag, that may be collapsed axially, to form a compactly folded cylinder. The film used to form the bag may be chosen for its flexibility, ability to be thermoformed into a flexible shape, ability to be welded to other components, and/or odour barrier properties.

The collection device may be especially suitable for use in a controlled evacuation device, but it is not limited exclusively to this. Additionally, or alternatively, the collection device may be especially suitable for use in an ostomy appliance using a deployable collection device, but the invention is not limited exclusively to this.

### Brief Description of the Drawings

Non-limiting embodiments of the invention are now described, by way of example only, with reference to the accompanying drawings, in which:
Figs. 1A and 1B are schematic sections showing a controlled evacuation ostomy appliance employing a deployable collection device, in its blocking and non-blocking conditions, respectively;
Figs. 2A, 2B and 2C are schematic views showing a first embodiment illustrating the principle of vacuum forming the collection device from plastics sheet, and Figs. 2D, 2E and 2F are schematic views illustrating other examples of Forms;
Fig. 3 is a schematic section illustrating a second embodiment of vacuum forming;
Fig. 4 is a schematic section illustrating a third embodiment of vacuum forming;
Figs. 5A and 5B are schematic sections showing a first example of collapsible Form in its compact and deployed conditions, respectively, and Fig. 5C is a schematic perspective view of a collection device formed by the Form;
Figs. 6A and 6B are schematic sections showing a second example of collapsible Form in its compact and deployed conditions, respectively;
Fig. 7A is a schematic perspective view showing a coupling ring pre-attached to a sheet before vacuum forming, and Fig. 7B is a schematic section showing the Form during vacuum forming;
Fig. 8A is a schematic section showing integral attachment of a plastics member to a sheet during vacuum forming, and Figs. 8B-E are schematic perspective views showing the integral attachment process;
Figs. 9A and 9B are schematic sections illustrating vacuum forming using a female Form;
Figs. 10A and 10B are schematic side and front perspective views of a first example of pouch including a front vacuum formed portion; and
Fig. 11 is a schematic side view of a second example of pouch including front and rear vacuum formed portions.

### Detailed Description of Preferred Embodiments

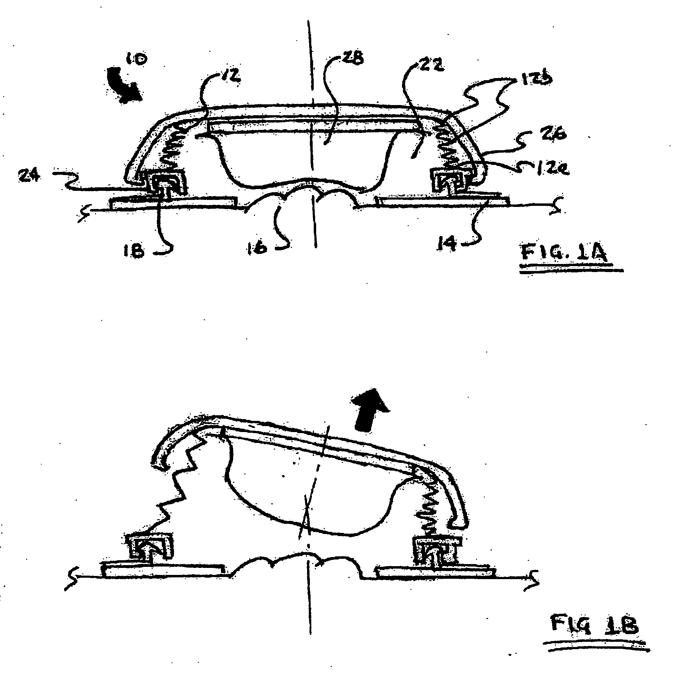

Referring to Fig. 1, an ostomy appliance 10 may be illustrated employing a deployable collection device 12 for body waste. In the present embodiment, the appliance 10 may be in the form of a controlled evacuation device for controlling discharge of body waste from the stoma, but the collection device 12 may equally be used in other ostomy appliances without controlled discharge. Also, in the present embodiment, the collection device 12 may be of a type that is deployable from a compact condition, to a deployed condition, but the collection device 12 need not be of such a "deployable" type in all embodiments.

The appliance 10 may generally comprise some of all of:
(a) an adhesive faceplate 14 for attachment to the wearer's skin around a stoma 16, and carrying a first coupling ring 18;
(b) the collection device 12 having a proximal end 12a and a distal end 12b. The collection device may comprise at least one aperture 20, for example, at the proximal end. The collection device 12 may optionally comprise a second aperture 22, for example, at the distal end;
(c) an intermediate coupling ring 24 mateable with the first coupling ring 18, and attached to the proximal end 12a of the deployable collection device 12;
(d) a cover 26 mateable with, or removeably attached to, the intermediate coupling ring 24, and attached to the distal end 12b of the deployable collection device 12; and
(e) a stoma seal 28 for creating a seal in or against the stoma 16 for obstructing the discharge of at least solid body waste from the stoma 16. The seal 28 may be carried directly or indirectly by the cover 26.

The appliance may have two operating conditions. The first, illustrated in Fig. 1A, may be the blocking (or non-discharge) condition, in which the cover 26 may be mated to or attached to the intermediate coupling ring 24, such that the collection device 12 may be held in a stowed condition between the cover 26 and the intermediate coupling ring 24, and the seal 28 may be positioned such that it may obstruct discharge from the stoma 16. The blocking condition may be the condition in which the appliance 10 may initially be supplied to, and worn by, a user. The second condition, illustrated in Fig. 1B, may be the non-blocking (or discharge) condition, which the user selects when the user desires to discharge waste from the stomal. In the second condition, the cover 26 may be separated from, and displaced away from, the intermediate coupling ring 24 in order to remove the seal 28 from the stoma, and to distend the collection device 12 to a deployed condition. In the deployed condition, the collection device 12 may provide a substantial collection volume for collecting and containing stomal discharge.

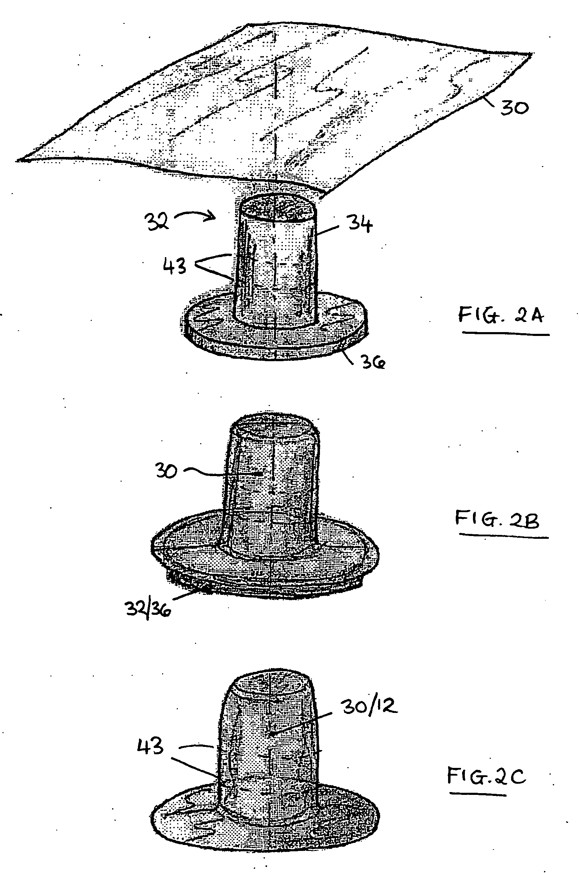

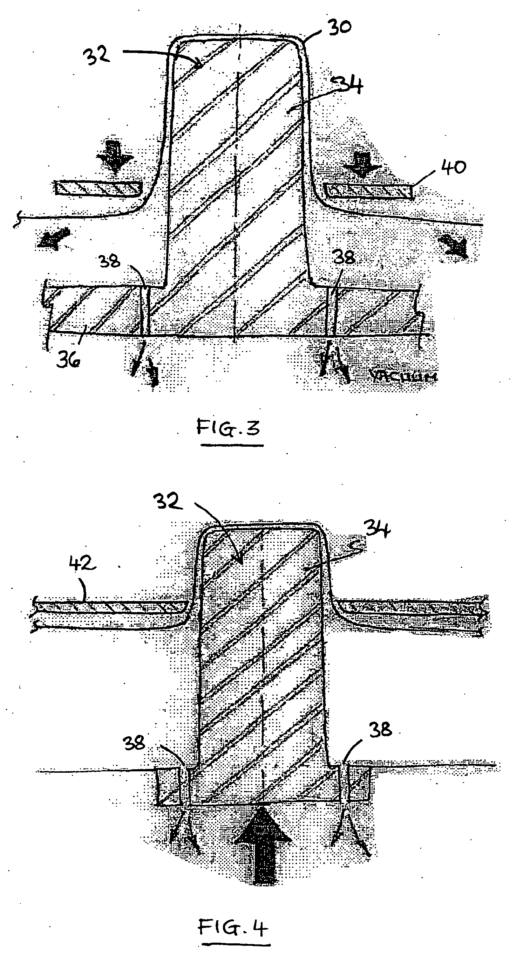

Referring to Figs. 2A, 2B, 2C and 3, the collection device 12 may generally comprise a thermoformed bag, made of flexible plastics material. Hereafter, the terms collection device and bag may be used interchangeably (although the term bag may be used generally to describe the article during manufacture, and the term collection device may be used to describe the article after manufacture). The especially preferred thermoforming techniques for which the invention is especially suitable may be vacuum forming and/or pressure forming. However, other thermoforming techniques may be used that generate a pressure differential on either side of a sheet in order to draw the sheet against a shape defining Form. The following examples describe vacuum forming, but the same principles may be used in other thermoforming techniques.

The bag may be vacuum formed in its deployed shape. Thereafter, the bag may be collapsed into its stowed shape for use in the appliance 10. The bag may have a generally hollow tubular shape, although other shapes may be used as desired. The use of the thermoforming process described herein may enable the collection device 12 (in its deployed shape) to have a depth-to-diameter ratio greater than 1:1, for example, at least 2:1, or at least 3:1, or at least 4:1, or at least 5:1, or at least 6:1, or at least 7:1, or at least 8:1, or at least 9:1, or at least 10:1.

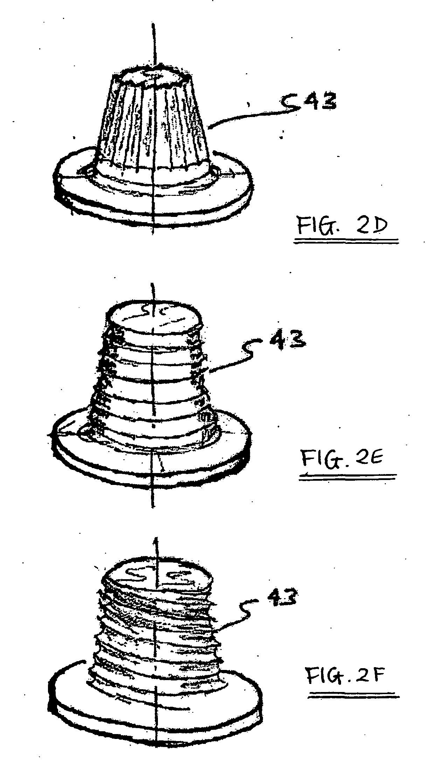

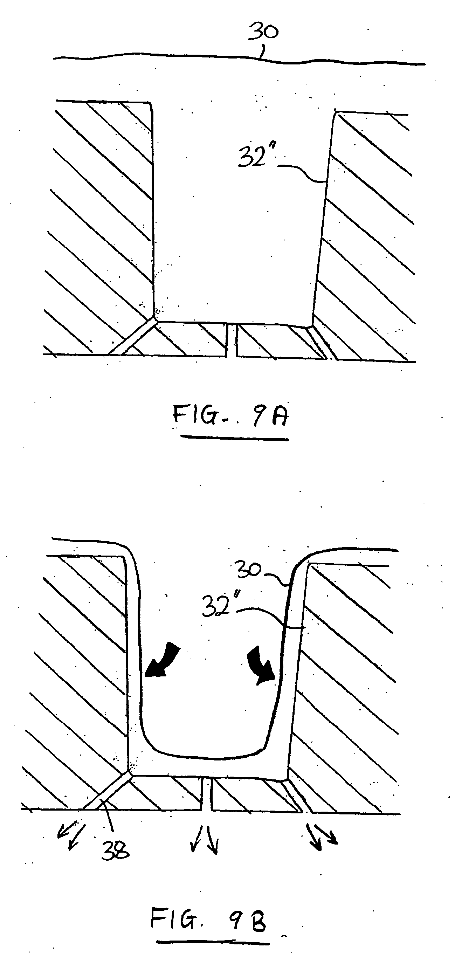

The bag may be vacuum formed from a sheet (or film) 30 of flexible plastics material, vacuum drawn against a Form 32. In Fig. 2, the Form 32 may be a male Form, although a female Form may be used as desired (and described later with respect to Fig. 9). The Form 32 may comprise an upstand 34 projecting from a base 36. One or more suction ports 38 may be formed in the upstand 34 and/or the base 36, for applying a vacuum in order to draw the plastics sheet 30 against the Form 32.

One issue that may be encountered when vacuum forming the collection device 12 with a high aspect ratio shape, may be the formation of pleats and wrinkle imperfections in the bag. These imperfections may result from the formation of pleats and wrinkles in the sheet 30 as the sheet 30 is drawn towards the bottom of the Form 32. Such imperfections may be undesirable aesthetically and/or technically, for example, if the imperfections may interfere with subsequent processing of the bag after vacuum forming. The formation of pleats and/or wrinkles may optionally be controlled or substantially eliminated, by using a female structure, such as a washer structure 40 (Fig. 3). The washer structure 40 may have an inner diameter that is slightly larger than the outer diameter of the upstand 34. The washer structure 40 may be placed over the sheet 30 on the Form 32 prior to applying the vacuum to the Form. The washer structure 40 may cause the sheet 30 to stretch and drape more evenly around the Form. Alternatively, referring to Fig. 4, the washer structure 40 may be replaced by an apertured plate 42. The apertured plate 42 may remain generally stationary, and the sheet 30 may be placed generally flat against the aperture plate 42. The upstand 34 of the Form 32 may be advanced against the sheet 30, at a position generally in register with the aperture in the plate 42, to displace the film through the aperture and around the Form 32. The diameter of the aperture may be slightly larger than the diameter of the upstand 34. The plate 42 may guide and stabilize the sheet 30 in a similar manner to the washer structure 40 described above.

The upstand 34 may have a generally smooth exterior surface. The vacuum formed bag may then have generally smooth shaped walls (subject to any wrinkling and pleating as explained above). When the collection device 12 may be collapsed to its compact form, the side wall of the bag may collapse generally randomly, along random fold lines.

Alternatively, as indicated in phantom at 43 in Figs. 2A-C, the exterior surface may include one or more surface features for imparting a corresponding shape feature to the vacuum formed bag. Such a shape feature may stabilize the bag during collapsing, such that the bag can collapse in a predetermined way and/or with folds at predetermined positions and/or along predetermined fold lines. The shape features may include axial features and/or circumferential features and/or spiral features. The shape features may appear to have a random pattern, or a degree of uniformity or non-randomness. By way, of example, the shape features may include one or more circumferential ribs (projecting or recessed), or folds. The shape features may be small, such that they do not interfere with removal of the Form 32 from the vacuum formed bag. Alternatively, if larger shape features may be desired, then an expanding Form 32' may be suitable (as described later). Figs. 2D-F may illustrate example Forms for creating such surface shape features.

The upstand 34 of the Form 32 may be of generally parallel cylindrical shape. The bag formed using such a Form 32 may be of about equal diameter along its entire length, such that the ends are of about the same diameter as each other (subject to possible inward rounding at the end of the bag corresponding to the tip of the upstand 34, and possible outward rounding at the end of the bag corresponding to the point at which the upstand 34 meets the base 36).

Alternatively, the upstand 34 may have a narrowing shape, such that the upstand 34 narrows in a direction from the base 36 to the tip of the upstand 34. The upstand 34 may narrow progressively and/or with one or more steps. Such a shape of Form 32 may produce a bag having one end (e.g. the distal end 12b) adjacent to the tip of the upstand 34 narrower in diameter than the opposite end (e.g. the proximal end 12a) adjacent to the base 36. Such a narrowing shape may be beneficial in aiding removal of the bag from the Form 32 after vacuum forming.

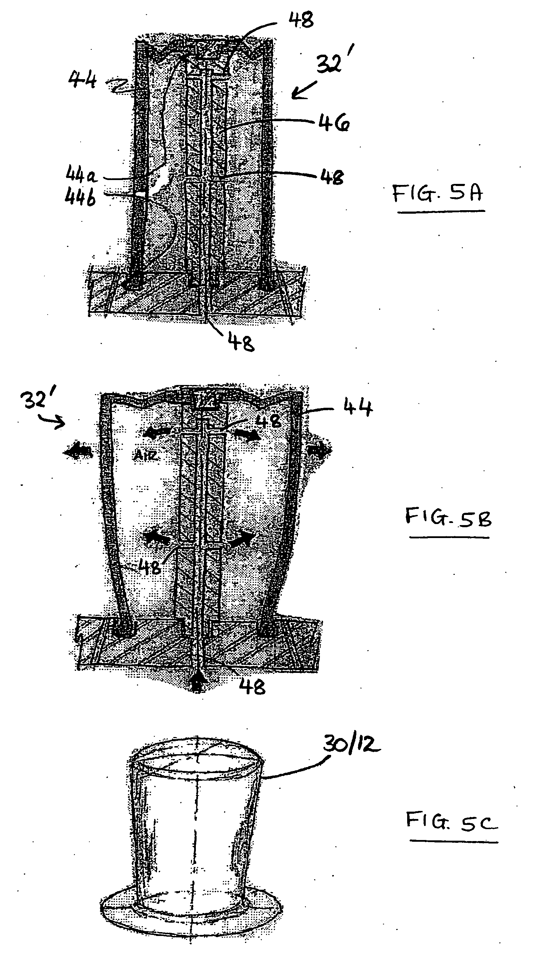

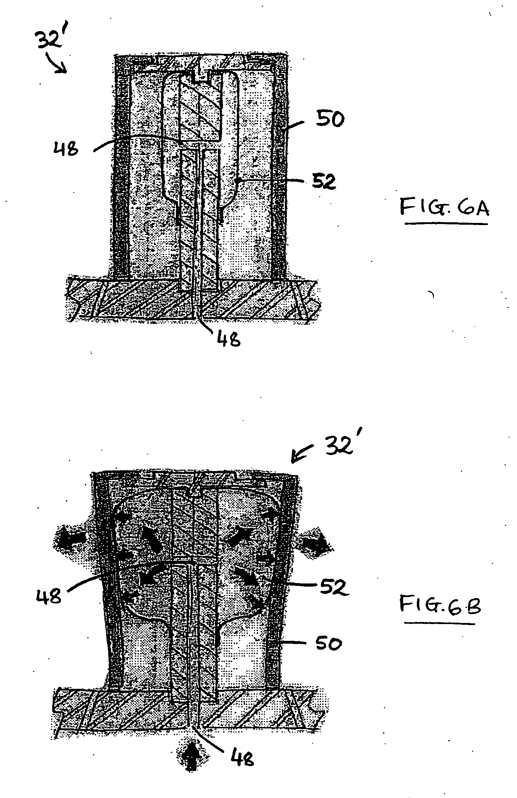

In other cases, it may be desired to create a bag having shape in which the diameter at a first arbitrary point at a first distance from the base 36 is larger than the diameter at a second arbitrary point at a second distance from the base 36 larger than the first distance. Such a bag might be formed by a Form 32 of undercut shape. It might be difficult to form such a bag shape using a fixed-shape Form 32 without causing undesirable expansion of the bag when the bag is removed from the Form 32 (or vice versa). In such a case, an expanding Form 32' may be appropriate, as illustrated in Figs. 5 and 6. The term "expanding" Form may be used herein to refer to any Form 32' that is capable of changing shape between a deployed shape (e.g. the shape of the Form for the vacuum forming process), and a compacted shape (e.g. a smaller shape to allow easy removal of the Form 32' from within the vacuum formed bag). The expanding Form 32' may have a natural shape with respect to which the expanding Form 32' is capable of resilient deformation. For example, the natural shape could be either of the deployed and compacted shapes, or an intermediate shape.

Referring to Fig. 5, a first example of expanding Form 32' may be comprise an integral, elastic, inflatable Form member 44 that is sealed at both ends 44a and 44b. The Form member 44 may be supported by a central column 46 projecting from the base 36. The natural shape of the Form member 44 (Fig. 5a) may correspond to its contracted shape. The Form member 44 may be pressurized via inflation ports 48, to expand the Form member 44 to its deployed shape (Fig. 5b). The deployed shape may be an inverted, smoothly tapered frusto-cone, that narrows in diameter from its tip to the base 36. After the bag has been vacuum formed around the Form member 44 in the deployed shape of the Form member 44 (Fig. 5c), the Form member 44 may be depressurized to allow the Form member to return to its contracted shape, to allow the vacuum formed bag to be easily removed from the Form 32', without substantially expanding or distorting the shape of the vacuum formed bag.

Referring to Fig. 6, a second example of expanding Form 32' may comprise an external Form skin 50, and an internal expansion bladder 52. In a similar manner to the first example of Fig. 5, the natural shape of the Form skin 50 may be its contracted shape. By applying a pressure via the inflation ports 48, the internal inflation bladder 52 may be pressurized to deform the Form skin 50 outwardly to its deployed shape. When the inflation pressure may be removed, the Form skin 50 may be allowed to return elastically to its contracted shape.

Suitable materials for the Form 32' may include elastomers that are typically used in industrial and medical product applications. These may include vulcanized rubbers such as BUNA and Viton, silicone rubbers, and certain thermoplastic elastomers. It is also possible to create an expanding/contracting Form 32' using a flexible, non-elastic material. By virtue of the shape and material properties, such a Form 32' may be expanded and relaxed at appropriate times. In the relaxed state, the material may be sufficiently flexible to allow easy removal of the vacuum formed bag. Thin, flexible, heat resistant materials, such as mylar may be suitable for such a Form 32'.

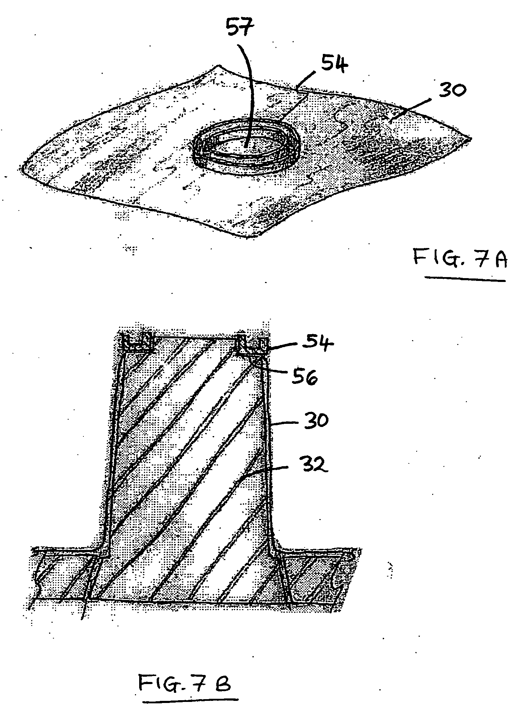

In general, other components or accessories may be attached to the bag, either as part of the vacuum forming process, or as an additional step before or after vacuum formation. For example, referring to Fig. 7(a), a thermoplastic coupling ring 54 (which may be the intermediate coupling ring 24) may be attached to the sheet 30 prior to vacuum forming. The Form 32 may include a recess 56 (Fig. 7b) for at least partly accommodating the coupling ring 54. The coupling ring may serve to positively locate the sheet 30 with respect to the Form 32. The portion 57 of the sheet material 30 inside the coupling ring 54 may be removed either before or after the vacuum forming process. If the portion 56 of material is removed before vacuum forming, the coupling ring 54 may provide a seal between the sheet 30 and the Form 32, so that a vacuum may still be applied to the remainder of the sheet 30.

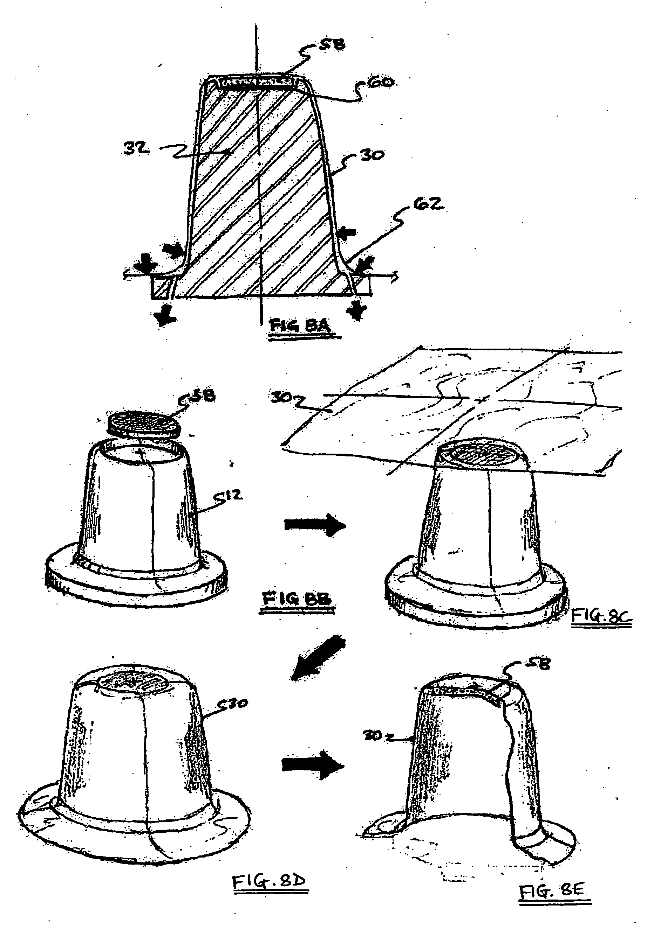

Figs. 8A-E may illustrate integral attachment of a thermoplastic component 58 during the vacuum forming process. The thermoplastic component 58 may be positioned in, against or around, the Form 32 before the sheet 30 is draped over the Form. The Form 32 may include a recess 60 for at least partly accommodating the component 58. During the vacuum forming process, the sheet 30 may be forced into contact with the component 58, and may bond thereto by virtue of heat and the pressure generated by the vacuum forming process. Although, the component 58 may be illustrated in Fig. 8 as being positioned at the distal end of the bag, this is merely by way of example. The component 58 may be positioned at any desired location, for example, at the open end of the bag. Such a location may be suitable for integrally attaching the intermediate coupling ring 24 of the appliance 10.

Alternatively, a component may be attached to the bag after the vacuum forming process. For example, a mouth portion 62 (Fig. 8A) of the bag corresponding to the junction between the upstand 34 and the base 36 may typically include a peripheral flange or other outward facing planar surface. Such a flange may be suitable as a planar surface for attaching to another component (e.g. the intermediate coupling ring 24) or to another sheet.

A preferred method of attaching the collection device 12 to another component (such as the intermediate coupling ring 24 or the cover 26) may be by welding. Such welding may include any of integral welding (as part of the vacuum formation process), heat welding, radio frequency welding, or laser welding. However, other attachment techniques, such as adhesive bonding, may be used as desired.

The sheet 30 may made of any suitable material, or combination (e.g. laminate) of materials that can be vacuum formed as desired in the present invention. The sheet 30 may have odour barrier properties for obstructing transpiration of malodours through the plastics material. Additionally, or alternatively, the material may be selected so as to have desired weldable properties, for example, so as to be weldable to other components of the appliance 10.

Suitable plastics materials (with good welding and odour barrier properties) may, by way of example only, include low density polyethylene (LDPE), ethylene vinyl acetate (EVA), and multi-layer films, such as ethylene-vinyl-acetate/poly-vinylidene-chloride/ethylene-vinyl-acetate (EVA / PVDC / EVA). Ethylene vinyl alcohol may be substituted for PVDC if desired. Many other single or multiple layer films may be used as desired, such as polypropylene and/or nylon. The material may be at least partly biodegradable.

The sheet or film 30 may have a thickness of at least about 0.01 mm. Additionally or alternatively, the thickness may be less than or equal to about 5mm, or less than or equal to about 2mm.

After vacuum forming, the bag may be trimmed of excess sheet material, to leave only the material defining the desired bag shape of the collection device 12. The collection device 12 may be collapsed from its originally formed shape to its compact shape, ready for storage or for assembly into the appliance. The collection device 12 may be collapsed around a mandrel or other former inserted into the collection device 12. Use of the mandrel can ensure that the cylindrical side wall of the collection device 12 does not collapse into the central space that is to be occupied by the seal 28.

Although the embodiments of Forms 32 and 32' described above have been illustrated as a male Form, the same principles may be applied using a female Form 32", for example as illustrated in Fig. 9. The female Form 32" may implement any of the male Form features described above. A female Form 32" may be especially suitable for forming bag shapes that may be difficult or even impossible to create using a male Form. The choice of male of female Form can be made according to the properties of the desired collecting device 12, and the desired manufacturing criteria.

Although the collection device 12 has been illustrated in the foregoing examples to have a circular shape, the above embodiments may use any closed-loop shape as desired, for example, a rectilinear shape. The examples hereafter may illustrate oval or egg-shaped forms.

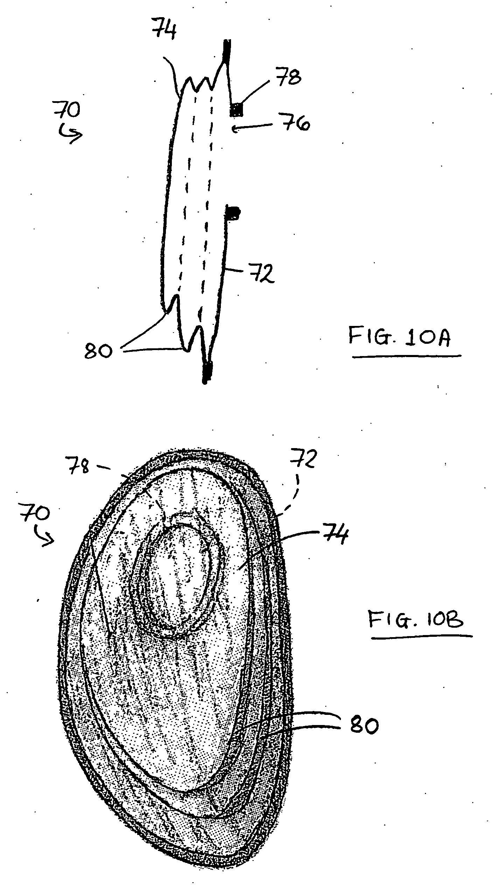

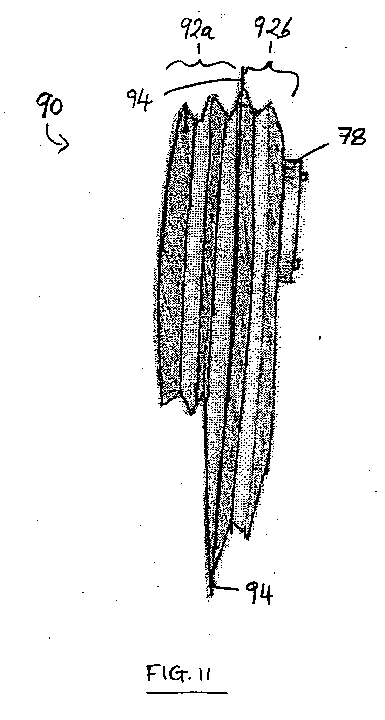

Also, although the principles of the invention have been illustrated in connection with a deployable collection device 12 for a controlled evacuation appliance 10, the invention is by no means limited to such an appliance. Figs. 10 and 11 may illustrate ostomy pouches 70 and 90 having collection devices incorporating vacuum formed portions.

Referring to Fig. 10, the ostomy pouch 70 may comprise a generally flat, conventional rear wall 72, and a thermoformed portion 74 forming the front wall of the pouch 70. The rear wall 72 may include a stomal aperture 76 and a conventional attachment device 78 for attaching the pouch 70 to a wearer's body. The thermoformed portion 74 may comprise concertina segments 80.

Referring to Fig. 11, thermoformed portions 92a and 92b may be used to implement both the front and rear "walls" of the pouch 90. In this embodiment, the entire collection chamber (collection device) of the pouch 90 may be thermoformed, and the concept of front and rear "walls" may be referred to here merely to relate the pouch 90 to a conventional ostomy pouch comprising generally flat, flexible, front and back walls. The thermoformed portions 92a and 92b may be vacuum formed as separate front and rear shells which are attached together along a mutual seam 94 after thermoforming, as it might be awkward to create the bag shape of Fig. 11 using a single Form.

A pouch 70 or 90 including at least a thermoformed portion 74, 92a or 92b, may offer one or more of the following advantages:
(a) the thermoformed portion 74 or 92 may be collapsed into a compact shape, so that the pouch 74 or 92 may initially be not substantially greater than a conventional ostomy pouch.
(b) the thermoformed portion 74 or 92 can expand to provide a pouch of substantial collection capacity.
(c) the thermoformed portion 74 or 92 can provide an expandable portion that can expand more efficiently and effectively than a conventional pouch wall.
(d) the thermoformed portion 74 or 92 can provide a region of controlled shape expansion, instead of uncontrolled deformation of a conventional pouch wall.
(e) the thermoformed portion 74 or 92 can be shaped to define a specific contour, such as one or more progressively smaller shapes, or one or more progressively larger shapes.

The shape of the pouch 70 or 90 may additionally be controlled, to provide one or more regions of controlled expansion, by welding specific areas of facing pouch film and/or specific areas of the vacuum formed material, to one another, or by attaching these facing areas to each other by means of low-tack adhesive to create designated zones of controlled expansion.

It will be appreciated that the use of a thermoformed (e.g. vacuum formed, or pressure-formed) collection device (portion) as in the present invention can enable an expandable collection device (portion) to be manufactured from sheet material, that can have a desirably small compact shape, yet also provide a substantial collection volume when desired.

The foregoing description is merely illustrative of preferred forms of the invention. Many modifications, alternatives and equivalents may be used within the scope of the invention.

## Claims

1. A method of producing at least a first portion of a collection device for an ostomy appliance wherein the first portion is deployable in use from a compact shape to a deployed shape, the method comprising:
providing a sheet of flexible plastics material (30); and
thermoforming by vacuum forming or pressure forming said first portion from the sheet of flexible plastics material (30) in a shape corresponding to said deployed shape.

2. The method according to claim 1, further comprising integrally attaching a second portion (54; 58; 92b) to the first portion during the step of thermoforming the first portion.

3. The method according to claim 2, wherein the second portion is selected as one 3. or more of: a second collection device portion (92b); a molded component (58); a coupling member (54); a flexible sheet.

4. The method according to any of claims 1 to 3, wherein the thermoforming is performed using a male Form (32) and a female structure (40), the female structure comprising an aperture dimensioned to receive the male Form with only narrow gap therebetween, and wherein the method comprises moving at least one of the male Form and the female structure relative to the other, whereby the female structure smoothes the sheet around the male Form.

## Patentansprüche

1. Verfahren zur Herstellung wenigstens eines ersten Teiles einer Sammelvorrichtung für eine Anwendung bei einer Stomaeinrichtung, bei der der erste Teil bei Gebrauch aus einer kompakten Gestalt in eine entfaltete Gestalt entfaltbar ist und wobei das Verfahren umfasst:
das Bereitstellen einer Folie aus einem flexiblen Kunststoffmaterial (30), und
das Thermoformen durch Vakuumformen oder Druckformen des besagten ersten Teiles aus der Folie aus flexiblem Kunststoffmaterial (30) in eine Gestalt, die der besagten entfalteten Gestalt entspricht.

2. Verfahren gemäß Anspruch 1, ferner umfassend ein integrales Befestigen eines zweiten Teiles (54, 58, 92b) am ersten Teil während des Schrittes des Thermoformens des ersten Teiles.

3. Verfahren gemäß Anspruch 2, wobei der zweite Teil aus einem oder mehreren der folgenden Teile ausgewählt wird: ein zweiter Teil einer Sammelvorrichtung (92b), ein Formteil (58), ein Kupplungselement (54), eine flexible Folie.

4. Verfahren gemäß einem der Ansprüche 1 bis 3, wobei das Thermoformen unter Verwendung einer männlichen Form (32) und einen weiblichen Struktur (40) durchgeführt wird, wobei die weibliche Struktur eine Öffnung dimensioniert, um die männliche Form mit einem nur schmalen Spalt zwischen beiden aufzunehmen, und wobei das Verfahren das Bewegen wenigstens entweder der männlichen Form oder der weibliche Struktur relativ zur jeweils anderen umfaßt, wobei die weibliche Struktur das Blatt um die männliche Form herum glättet.

## Revendications

1. Procédé de production d'au moins une première partie d'un dispositif de collecte pour un appareillage stomique où la première partie peut être déployée lors de l'utilisation d'une forme compacte à une forme déployée, le procédé comprenant le fait :
de fournir une feuille de matériau plastique souple (30) ; et
de thermoformer par formage sous vide ou par formage à la presse ladite première partie à partir de la feuille de matériau plastique souple (30) sous une forme correspondant à ladite forme déployée.

2. Procédé selon la revendication 1, comprenant en outre le fait de fixer de manière solidaire une deuxième partie (54 ; 58 ; 92b) à la première partie pendant l'étape de thermoformage de la première partie.

3. Procédé selon la revendication 2, dans lequel la deuxième partie est choisie comme un ou plusieurs éléments parmi : une deuxième partie de dispositif de collecte (92b) ; un composant moulé (58) ; un élément de couplage (54) ; une feuille souple.

4. Procédé selon l'une quelconque des revendications 1 à 3, dans lequel le thermoformage est effectué en utilisant une forme mâle (32) et une structure femelle (40), la structure femelle comprenant une ouverture dimensionnée pour recevoir la forme mâle avec seulement un espace étroit entre celles-ci, et où le procédé comprend le fait de déplacer au moins l'une de la forme mâle et de la structure femelle par rapport à l'autre, moyennant quoi la structure femelle lisse la feuille autour de la forme mâle.
